# EUROPÄISCHE PATENTSCHRIFT

(11) **EP 3 139 975 B1**
(45) Veröffentlichungstag und Bekanntmachung des Hinweises auf die Patenterteilung: **28.08.2019**
(21) Anmeldenummer: 15721576.5
(22) Anmeldetag: 07.05.2015
(51) Int. Cl.: A61M 1/16

(54) **VERFAHREN ZUR BEFÜLLUNG EINES BEHÄLTERS**
METHOD FOR FILLING A CONTAINER
PROCÉDÉ DE REMPLISSAGE D'UN RÉCIPIENT

(30) Priorität: 08.05.2014 DE 102014006821
(43) Veröffentlichungstag der Anmeldung: 15.03.2017
(73) Patentinhaber: Fresenius Medical Care Deutschland GmbH, 61352 Bad Homburg (DE)
(72) Erfinder: KLÖFFEL, Peter, 97720 Nüdlingen (DE); NICHOLAS, Olaf, 97318 Kitzingen (DE)
(74) Vertreter: Herrmann, Uwe
(86) Internationale Anmeldenummer: PCT/EP2015/000929
(87) Internationale Veröffentlichungsnummer: WO 2015/169445

(56) Entgegenhaltungen:
- EP-A1- 1 393 761
- DE-A1-102011 106 248
- DE-C1- 10 201 109
- DE-C1- 19 728 800

## Beschreibung

Die vorliegende Erfindung betrifft ein Verfahren zur Befüllung eines Behälters vorzugsweise enthaltend wenigstens ein Konzentrat, wobei das Konzentrat derart ausgebildet ist, dass es bei seiner Lösung oder bei seiner Verdünnung in/mit einer Flüssigkeit, vorzugsweise Wasser, wenigstens ein Flüssigkonzentrat oder einen Teil eines Flüssigkonzentrats bildet, das zur Herstellung wenigstens einer Dialyselösung geeignet ist, und wobei die Befüllung des Behälters mittels des Bilanzkammersystems eines Dialysegerätes erfolgt, das Kammern aufweist, aus denen die Flüssigkeit in Form von sich wiederholenden Zyklen in den Behälter gefördert wird.

Aus dem Stand der Technik ist es bekannt, eine Dialyselösung mit Hilfe von Trockenkonzentraten herzustellen. Diese Trockenkonzentrate enthalten beispielsweise verschiedene Salze, die durch Wasser in Lösung gebracht werden. Die auf diese Weise hergestellte konzentrierte Lösung wird verwendet, um ggf. mit weiteren Konzentraten und Wasser eine gebrauchsfertige Dialyselösung herzustellen, die dann im Rahmen eines Dialyseverfahrens eingesetzt wird.

Um Wasser in den das Trockenkonzentrat enthaltenden Behälter einzuleiten, kann eine Pumpe oder beispielsweise auch das Bilanzkammersystem des Dialysegerätes verwendet werden. Eine solche Vorgehensweise sowie ein Trockenkonzentrat enthaltender Behälter ist beispielsweise aus der DE 10 2011 106 248 A1 bekannt. Ein Bilanzkammersystem dient während der Durchführung einer Dialysebehandlung zur Steuerung des dem Dialysator zugeführten und des von dem Dialysator abgeführten Volumens der Dialyselösung.

Das Bilanzkammersystem besteht üblicherweise aus zwei starren Kammern mit festem Volumen, die jeweils durch eine bewegliche Trennmembran in ein Abteil für frische Dialyselösung (im Folgenden auch als "Frischwasserabteil" bezeichnet), die bei der Behandlung zum Dialysator hin geführt wird, und in ein Abteil für verbrauchte Dialyselösung (im Folgenden auch als "Altwasserabteil" bezeichnet), die bei der Behandlung vom Dialysator in die Kammer strömt, getrennt wird. Während der Behandlung wird von beiden Kammern Flüssigkeit gefördert, nämlich frische Dialyselösung aus einer Quelle für die Dialyselösung in den Dialysator und verbrauchte Dialyselösung vom Dialysator in den Abfall.

Aufgrund der Bewegung der Trennmembran in der Kammer und der Betätigung von Ventilen, die stromaufwärts und stromabwärts der Kammern angeordnet sind, ist das Volumen, das vom Dialysator weg gefördert wird, identisch zu dem Volumen, das zum Dialysator hin gefördert wird.

Wird ein solches Bilanziersystem des Dialysegerätes zur Füllung eines Konzentratbehälters verwendet, wird keine verbrauchte Dialyselösung von Dialysator in das Bilanziersystem eingeleitet.

Bei der Füllung des Behälters mittels der Kammern des Bilanzkammersystems ist darauf zu achten, dass das Gesamtfüllvolumen den Vorgaben entspricht, damit sichergestellt ist, dass die gewünschte Füllmenge erreicht wird bzw. dass das Trockenkonzentrat tatsächlich gelöst wird bzw. das das korrekte Mischungsverhältnis eingehalten wird und somit die gewünschten Konzentrationen der gelösten Stoffe in dem hergestellten flüssigen Konzentrat erhalten werden.

Soll das Gesamtfüllvolumen, d.h. das insgesamt in den Behälter geförderte Flüssigkeitsvolumen aus der Anzahl der Füllzyklen und dem geförderten Füllvolumen pro Zyklus bestimmt werden, ist somit sicherzustellen, dass das pro Zyklus geförderte Füllvolumen den Vorgaben entspricht bzw. bekannt ist. Tritt eine Leckage bzw. eine Undichtigkeit beispielsweise an einem Ventil auf, das eine Kammer des Bilanzkammersystem abschließt, hätte dies zur Folge, dass das Füllvolumen, das dem Behälter pro Zyklus zugeführt wird und somit das Gesamtfüllvolumen nicht dem Sollwert entspricht, was selbstverständlich unerwünscht ist. Denn das Auflösen des in dem Behälter befindlichen Granulats ist ein wichtiger Aspekt in der Konzentrataufbereitung. Messungen haben gezeigt, dass sich das Granulat üblicher Behälter ab einem Füllvolumen von 3,6 I komplett auflöst. Um dieses Füllvolumen sicherzustellen, lassen sich Grenzwerte, d.h. Toleranzen beispielsweise in der Höhe von 50 µl/Umschattung festlegen, die nicht überschritten werden dürfen.

Wird dieser Grenzwert des Füllvolumens gleichwohl z.B. durch eine Undichtigkeit überschritten, hätte dies unter Umständen ein ungenügendes Auflösen des Trockenkonzentrats und somit Konzentrationen der Stoffe in dem flüssigen Konzentrat zur Folge, die außerhalb der vergleichsweise engen Spezifikationsgrenzen liegen. Entsprechendes gilt bei der Verwendung von flüssigen Konzentraten. Auch bei diesen ist sicherzustellen, dass die durch Mischen erhaltene Lösung die gewünschten Konzentrationen aufweist.

In der US 4,431,425 ist eine Vorrichtung zur Leckageerkennung der beweglichen Trennwand einer Membranvorrichtung zur Förderung einer Infusionslösung mit einem optischen Sensor beschrieben. Die DE 197 28 280 C1 offenbart eine Vorrichtung zum Fördern von Flüssigkeiten für eine medizinische Behandlungsvorrichtung, bei der die Anzahl der Druckimpulse über die Zeit überwacht wird.

Die standardmäßig durchgeführten Druckhaltetests der Ventile des Bilanzkammersystems und des angeschlossenen Leitungssystems sind nicht empfindlich genug, so dass ein empfindlich reagierendes Überwachungsverfahren für die Dichtigkeit des Systems notwendig ist.

Der vorliegenden Erfindung liegt somit die Aufgabe zugrunde, ein Verfahren bereitzustellen, mittels dessen zuverlässig erkannt werden kann, ob eine Undichtigkeit des Bilanzkammersystems oder des mit diesem in Verbindung stehenden Leitungssystems vorliegt, durch die das in den Behälter geförderte Fördervolumen verringert wird.

Diese Aufgabe wird durch ein Verfahren mit den Merkmalen des Anspruchs 1 gelöst. Danach ist vorgesehen, dass der zeitliche Verlauf des Druckes während eines Zyklus der Füllphase des Behälters gemessen wird und dass ein Alarmsignal ausgegeben wird und/oder der Füllvorgang des Behälters gestoppt wird, wenn der gemessene Maximaldruck in einem Zyklus einen Grenzwert nicht übersteigt. Der Behälter, der vor seiner Befüllung gemäß dem erfindungsgemäßen Verfahren leer sein kann oder Konzentrat enthalten kann, wird im Folgenden einfach als "Behälter" oder als "Trockenkonzentratbehälter" bezeichnet.

An dieser Stelle wird darauf hingewiesen, dass es sich bei dem Behälterinhalt nicht zwingend um ein Trockenkonzentrat handeln muss, wenngleich die Erfindung für die Auflösung von Trockenkonzentraten besondere Relevanz hat. Die Erfindung ist somit nicht auf das Auflösen von Trockenkonzentraten beschränkt, wenngleich diese eine bevorzugte Ausgestaltung der Erfindung darstellen. Bei dem Behälterinhalt kann es sich auch um ein flüssiges Konzentrat handeln, das durch die Zugabe von Flüssigkeit verdünnt wird oder auch um eine Mischung aus einem flüssigen und aus einem trockenen Konzentrat.

Auch ist von der Erfindung die Befüllung eines leeren und somit auch kein Konzentrat enthaltenden Behälters umfasst.

Unter dem Begriff "Zyklus" ist eine Zeitspanne zu verstehen, die das Entleeren eines Abteils einer Kammer umfasst. Ein solcher Zyklus umfasst vorzugsweise das Öffnen eines Ventils, durch das die Flüssigkeit aus dem Abteil der Kammer abläuft, das Entleeren dieses Abteils sowie das anschließende Schließen dieses Ventils. Dabei ist das Ventil, über das das Abteil mit frischer Flüssigkeit gefüllt wird, geschlossen. Während dieses Zyklus vollzieht sich in einem Abteil der anderen Kammer des Bilanzkammersystems der umgekehrte Vorgang. Dort wird das Ventil, durch das Flüssigkeit in das Abteil einläuft, geöffnet und das Abteil mit Flüssigkeit gefüllt, während das Ventil, durch das Flüssigkeit aus dem Abteil abläuft, geschlossen ist.

Der Zyklus ist beendet, wenn ein Abteil einer Kammer entleert wurde und ein Abteil der anderen Kammer gefüllt wurde. Durch eine Umschaltung der Ventile beginnt ein neuer Zyklus, der das Entleeren des gefüllten Abteils und das Füllen des leeren Abteils umfasst.

Ein Beispiel für die sich in Zyklen vollziehende Funktionsweise des Bilanzkammersystems findet sich in der DE 26 34 238 A1, auf die insoweit Bezug genommen wird.

Der vorliegenden Erfindung liegt somit der Gedanke zugrunde, eine Dichtigkeitsbestimmung über die Höhe des Drucks bzw. über die Größe der Druckimpulse und nicht über deren Anzahl vorzunehmen. Bei der Ausdrückphase des Füllvolumens aus der Kammer bzw. aus deren Abteil steigt der Druck zunächst an und fällt dann wieder ab, so dass ein Druckimpuls entsteht. Bei Erreichen oder Unterschreiten eines unteren Grenzwertes kann ein Umschalten bzw. das Einleiten eines neuen Zyklus zur Befüllung des Behälters erfolgen.

Die Druckmessung erfolgt derart, dass der Druck gemessen wird, unter dem die von der Kammer zu dem Behälter gelangende Flüssigkeit steht. Die Druckmessung kann an oder in der Kammer und/oder an oder in der Leitung zwischen Kammer und dem Behälter und/oder an oder in dem Behälter erfolgen.

Übersteigt der während eines solchen Druckimpulses oder sonstigen Druckverlaufes gemessene Druck nicht einen oberen Grenzwert, wird darauf rückgeschlossen, dass eine Leckage vorliegen muss. Dies kann zur Ausgabe eines entsprechenden Signals und/oder zum Abbruch des Füllprozesses des Behälters führen. Mit einer derartigen Vorgehensweise kann erkannt werden, ob der Behälter, der vorzugsweise als Beutel ausgeführt ist, mit dem korrekten Füllvolumen beschickt wird oder mit einem leckagebedingten falschen Füllvolumen. Durch das erfindungsgemäße Verfahren lässt sich eine Überwachung einer Abweichung des tatsächlich geförderten Füllvolumens von dem gewünschten Füllvolumen in einer Größenordnung von 50 µl/Zyklus erkennen.

Vorzugsweise weist das Bilanzkammersystem des Dialysegerätes wenigstens zwei Kammern auf und es wird zum Zwecke der Förderung der Flüssigkeit in den Behälter ein Pendelvolumen zwischen den beiden Kammern hin und her bewegt. Dieses Pendelvolumen legt das pro Bilanzkammerumschaltung geförderte Fördervolumen für den Trockenkonzentratbehälter fest. Über die Gesamtanzahl der Umschaltungen kann das Gesamtfördervolumen ermittelt werden, das dem Behälter zugeführt wurde. Das Pendelvolumen befindet sich vorzugsweise in dem Teil der Kammern, die im Behandlungsbetrieb mit verbrauchter Dialyselösung beaufschlagt werden, d.h. in den Altwasserabteilen. Die Förderung der Flüssigkeit in den Behälter erfolgt vorzugsweise mittels der Kammern, die im Behandlungsbetrieb die frische Dialyselösung zum Dialysator fördern, d.h. mittels der Frischwasserabteile.

Umfasst das Bilanzkammersystem zwei Kammern, ist das Pendelvolumen durch das Volumen von zwei Abteilen der Kammern des Bilanzkammersystems, vorzugsweise durch das Volumen der zwei Altwasserabteile der Kammern sowie durch die diese beiden Abteile miteinander verbindende Leitung einschließlich der darin befindlichen Ventile definiert.

Das erfindungsgemäße Verfahren findet vorzugsweise vor einer Dialysebehandlung statt. Von der Erfindung ist jedoch auch der Fall umfasst, dass das erfindungsgemäße Verfahren während einer Dialysebehandlung stattfindet, jedoch wird in diesem Fall der durch das erfindungsgemäße Verfahren befüllte Behälter nicht für die laufende Dialysebehandlung verwendet. Dessen Verwendung für eine Dialysebehandlung beginnt erst dann, wenn das erfindungsgemäße Verfahren zur Behälterbefüllung abgeschlossen ist und eine neue Behandlung erfolgt.

Das Pendelvolumen entspricht vorzugsweise dem Volumen einer gesamten Kammer des Bilanzkammersystems.

Tritt im System eine Undichtigkeit auf, kann dies zu einer Änderung des Pendelvolumens und somit auch zu einer Änderung des pro Zyklus geförderten Füllvolumens führen. Diese Undichtigkeit lässt sich mittels des erfindungsgemäßen Verfahrens ermitteln.

Vorzugsweise ist vorgesehen, dass der Grenzwert von dem Maximaldruck abhängt, der in wenigstens einem der vorhergegangenen Zyklen gemessen wurde. Wurde beispielsweise in einem der vorhergegangen Zyklen zur Befüllung des Behälters ein Maximaldruck von 400 hPa gemessen, kann vorgesehen sein, den Grenzwert für den oder die folgenden Zyklen in Abhängigkeit dieses Wertes zu bestimmen.

Für den ersten Zyklus kann der Grenzwert vorgegeben werden, da keine vorhergegangenen Zyklen existieren, anhand derer eine Grenzwertbestimmung erfolgen könnte.

Denkbar ist es, dass der Grenzwert um einen bestimmten Prozentsatz oder um einen bestimmten Absolutwert unter dem höchsten, in den vorhergegangenen Zyklen gemessenen Maximaldruck liegt. So kann der Grenzwert beispielsweise um 10 % unter dem gemessenen Maximaldruck liegen. Bezogen auf das angegebene Beispiel eines Maximaldruckes von 400 hPa beträgt der Grenzwert somit 360 hPa. Auch die Bestimmung des Grenzwertes durch Abzug eines festen Betrages, z.B. 50 hPa von dem Maximaldruck ist denkbar.

Bei diesen und im Folgenden genannten Werten handelt es sich nur um Beispiele, die die Erfindung nicht beschränken.

Vorzugsweise ist vorgesehen, dass es sich bei dem Maximaldruck um den höchsten Druck handelt, der in sämtlichen der vorhergegangenen Zyklen gemessen wurde. Liegen die maximalen Druckwerte in den vorhergegangen Zyklen beispielsweise in einer Spanne zwischen 390 hPa und 400 hPa, wird als Grundlage für die Bestimmung des Grenzwertes der größte Wert, in diesem Fall somit 400 hPa herangezogen.

Vorzugsweise ist vorgesehen, dass der Grenzwert im Laufe des Füllvorgangs nicht abgesenkt wird. Dies bedeutet, dass es bezogen auf das vorgenannte Beispiel bei einem Grenzwert von 360 hPa bleibt, selbst wenn sich in anderen, späteren Zyklen maximale Druckwerte unter 400 hPa ergeben sollte. In dieser Ausführungsform orientiert sich der Grenzwert somit nicht an dem zuletzt gemessenen Maximaldruck, sondern an dem größten Druckwert, der in einem der vorhergegangen Zyklen gemessen wurde.

Die Druckmessung kann beispielsweise in dem Leitungssystem zwischen der Kammer und dem Behälter oder in einer damit in Verbindung stehenden Leitung gemessen werden. Auch eine Druckmessung an oder in der Bilanzkammer oder an oder in dem Behälter selbst ist denkbar. Auch wenn der Behälter mit der Atmosphäre in Verbindung steht, ergibt sich durch den Strömungswiderstand des Schlauchsystems zwischen dem Bilanzkammersystem und dem Behälter ein charakteristischer Druckimpuls oder sonstiger Druckverlauf, der gemessen werden kann.

Vorzugsweise ist vorgesehen, dass der Druck bei einer Entleerung eines Abteils einer Kammer zunächst ansteigt und dann abfällt und dass ein Alarmsignal ausgegeben wird und/oder der Füllvorgang des Behälters gestoppt wird, wenn ein unterer Grenzwert für den Druck nicht innerhalb eines bestimmten Zeitfensters seit Zyklusbeginn erreicht oder unterschritten wird. So ist es beispielsweise denkbar, dass der Timeout, d.h. die maximal zulässige Zeitspanne bis zum Erreichen des unteren Grenzwertes 6 s (entsprechend einem Fluss von 300 ml/min) beträgt. Wird der untere Grenzwert in dieser Zeitspanne nicht erreicht, kann dies auf einen erhöhten Flusswiderstand im Behälter zurückgeführt werden. In diesem Fall kann der Füllvorgang abgebrochen werden und/oder es kann eine Meldung an den Nutzer ausgegeben werden. Diese Meldung besagt, dass der Behälter nicht gefüllt werden kann und dass ein neuer Behälter zu verwenden ist. Zusätzlich kann ein Fehlerspeichereintrag erfolgen. Dies kann entsprechend auch für den Fall gelten, dass der obere Grenzwert nicht erreicht wird.

Denkbar ist es somit, dass die Fehlerspeichereinträge lauten können:
1. Der Mindestdruck, d.h. der obere Grenzwert wurde nicht erreicht
2. Der untere Umschaltpunkt, d.h. der untere Grenzwert wurde nicht erreicht.

Weiterhin ist es denkbar, dass mehrere Timeouts definiert werden. So kann z.B. ein Timeout (z.B. 30 s) für den ersten Zyklus festgelegt werden, das länger ist als für alle folgenden Zyklen. Diese Vorgehensweise berücksichtigt, dass sich in der Rohrleitung in dem Behälter, durch die Flüssigkeit in den Behälter gefördert wird, ggf. noch Granulat bzw. Trockenkonzentrat vorliegt und dass diese Rohrleitung zunächst freigespült werden muss.

Denkbar ist es somit, dass ein oder mehrere Timeouts bzw. Zeitspannen vorgesehen sind, innerhalb derer der untere Grenzwert erreicht sein muss. Dabei kann das Zeitfenster für den ersten oder für die ersten Zyklen länger sein als für die folgenden Zyklen.

In einer weiteren Ausgestaltung der Erfindung ist vorgesehen, dass der erste oder die ersten Zyklen eines Füllvorgangs und/oder der letzte Zyklus vor einer Unterbrechung des Füllvorgangs nicht auf Erreichen oder Über- oder Unterschreiten eines Grenzwertes hin überwacht werden. Diese Vorgehensweise berücksichtigt das Einschwingverhalten des Systems zu Beginn des Füllvorgangs und nach einer Unterbrechung des Füllvorgangs.

Auch ist es denkbar, dass bei der Unterbrechung des Füllvorgangs der letzte Zyklus nicht überwacht wird. Der Grund dafür liegt darin, dass der Kompressor, der in einer Ausführungsform während des Füllvorgangs Druckluft oder ein sonstiges Gas in den Behälter einführt, bei einer Unterbrechung abgeschaltet oder dessen Luftstrom durch das Schließen eines Ventils gestoppt wird. Der sich daraus ergebende geringere Fluss zum Behälter hat zur Folge, dass sich in dem Behälter andere Druckverhältnisse ergeben, die ggf. zu einem Fehlalarm führen könnten. Der Abbruch wird dem System mitgeteilt. Die Variable wird beim Neustart des Füllvorgangs wieder gelöscht.

Wie bereits oben ausgeführt, ist es denkbar, dass der Behälter während des Füllvorgangs zur Atmosphäre hin belüftet ist. Gleichwohl ergeben sich aufgrund der Druckverluste in dem Leitungs- bzw. Schlauchsystem von der Kammer zum Behälter charakteristische Druckverläufe bzw. Druckwerte, die gemessen und ausgewertet werden können.

Wie diese ebenfalls oben ausgeführt wurde, ist vorzugsweise vorgesehen, dass der Grenzwert sich an dem im Rahmen eines Füllvorgangs eines Behälters bislang gemessen Maximaldruck orientiert, wobei vorzugsweise keine Verringerung des Grenzwertes erfolgt, wenn die Maximaldrücke über die Zeit fallen. Vorzugsweise ist ein Zurücksetzen des Grenzwertes nur dann vorgesehen und möglich, wenn bestätigt oder geräteseitig erkannt wird, dass ein neuer Behälter auf das Dialysegerät aufgesteckt wurde.

Bei dem Behälterinhalt kann es sich um einen beliebigen, in der Flüssigkeit löslichen Feststoff, beispielsweise um Pulver, um ein Granulat etc. oder auch um eine Flüssigkeit oder um eine fest/flüssig-Mischung handeln. Auch ist die Verwendung eines leeren bzw. nur mit Luft gefüllten Behälters denkbar. Vorzugsweise handelt es sich um ein saures Trockenkonzentrat, d.h. um ein Konzentrat, das nach dem Lösen in Wasser einen pH-Wert im sauren Bereich aufweist. Auch ein basisches Konzentrat, d.h. ein Konzentrat, das nach dem Lösen in Wasser einen pH-Wert im basischen Bereich aufweist, ist von der Erfindung umfasst.

An dieser Stelle wird darauf hingewiesen, dass der Begriff "Trockenkonzentrat" den Fall umfassen kann, dass sich ausschließlich Feststoffe und keine Flüssigkeit in dem Behälter befinden, jedoch auch den Fall, dass bereits flüssige Komponenten mit vorhanden sind, wie beispielsweise eine Säure etc.

Um eine hinreichende Durchmischung und damit eine Beschleunigung des Lösevorgangs/Mischvorgangs zu erreichen, kann vorgesehen sein, dass während oder zeitversetzt zu der Füllung des Behälters mit Flüssigkeit ein Gas, vorzugsweise Luft in den Behälter eingeleitet wird. Dazu kann der bereits oben gekannte Kompressor vorgesehen sein, der Luft in den Behälter einleitet. Die Luft kann über dasselbe Schlauchstück oder über dieselbe Leitung in den Behälter eingeleitet werden, wie die mittels des Bilanzkammersystems eingeleitete Flüssigkeit.

In einer weiteren Ausgestaltung der Erfindung ist vorgesehen, dass bei Erreichen eines unteren Grenzwertes für den Druck nur dann ein neuer Zyklus eingeleitet wird, wenn eine bestimmte Mindestzeitspanne seit Zyklusbeginn verstrichen ist. Denkbar ist es, dass der maximale Füllfluss z.B. auf einen Wert von 700 ml/min begrenzt ist. Wird der Umschaltdruck, d.h. der Druck, bei dessen Erreichen ein neuer Zyklus eingeleitet wird, früher erreicht, wird die Bilanzkammer in diesem Fall erst dann umgeschaltet, wenn auch die entsprechende Zeit für die Flussbegrenzung abgelaufen ist.

Um Wassermangelalarme bei schwachen RO-(Reversosmose)Anlagen zu vermeiden, ist der maximale Füllfluss an den Wasserzulauf gekoppelt.

Die vorliegende Erfindung betrifft des Weiteren ein Dialysegerät mit Mitteln zur Befüllung eines Behälters vorzugsweise enthaltend wenigstens ein Konzentrat, wobei das Konzentrat derart ausgebildet ist, dass es bei seiner Lösung oder bei seiner Mischung in/mit einer Flüssigkeit, vorzugsweise Wasser, wenigstens ein Flüssigkonzentrat oder einen Teil eines Flüssigkonzentrats bildet, das zur Herstellung wenigstens einer Dialyselösung geeignet ist, und wobei die Mittel zur Befüllung des Behälters wenigstens ein Bilanzkammersystem des Dialysegerätes umfassen, das Kammern aufweist, aus denen die Flüssigkeit in Form von wiederholten Zyklen in den Behälter bewegt wird, wobei das Dialysegerät wenigstens eine Steuer- oder Regelungseinheit aufweist, die ausgebildet und insbesondere programmiert ist, das Verfahren gemäß einem der Ansprüche 1 bis 13 durchzuführen.

Das Dialysegerät ist somit dazu geeignet und bestimmt, das erfindungsgemäße Verfahren auszuführen. Dazu dient eine Steuer- oder Regelungseinheit, die die Verfahrensschritte gemäß der vorliegenden Erfindung ausführt oder veranlasst.

Das Dialysegerät kann somit einen Alarmgeber und/oder Ventile aufweisen, der/die im Falle des nicht Erreichens des oberen Druckgrenzwertes ein z.B. optisches und/oder akustisches Signal an den Nutzer abgibt und/oder das oder die Ventile oder die Tätigkeit des Bilanzkammersystems schließt bzw. unterbindet, so dass der Füllvorgang des Behälters gestoppt wird.

Das Dialysegerät weist vorzugsweise Mittel auf, die derart ausgebildet sind, dass durch diese Mittel einer oder mehrere oder alle Verfahrensschritte gemäß der Erfindung ausgeführt werden können.

Weitere Einzelheiten und Vorteile der Erfindung werden anhand eines in der Zeichnung dargestellten Ausführungsbeispiels näher erläutert. Es zeigen:
- Figur 1:: ein schematisches Flussdiagramm eines Dialysegerätes gemäß der Erfindung,
- Figur 2:: den zeitlichen Verlauf des Druckes, des oberen Grenzwertes und der Anzahl der Füllzyklen während der Befüllung des Behälters in einem leckagefreien Fall und
- Figur 3:: den zeitlichen Verlauf des Druckes, des oberen Grenzwertes und der Anzahl der Füllzyklen während der Befüllung des Behälters bei Auftreten einer Leckage.

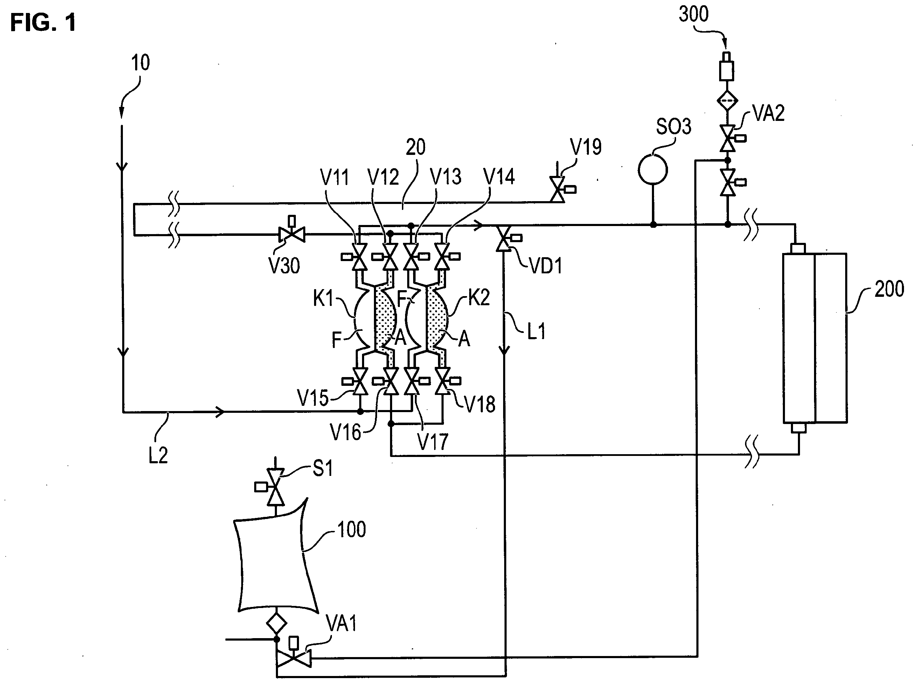

Aus Figur 1 ist ein Flussdiagramm eines Dialysegerätes gemäß der Erfindung dargestellt. Das Bezugszeichen 10 kennzeichnet einen RO-Wasserzulauf, von dem das Wasser zu dem Bilanzkammersystem 20 strömt.

Das Bilanzkammersystem 20 umfasst zwei parallel geschaltete Kammern K1 und K2, die beide starre Außenwandungen aufweisen und in denen sich jeweils eine bewegliche Membran erstreckt, die die Kammern K1 und K2 jeweils in zwei Bereiche, nämlich in ein Frischwasserabteil F und in ein Altwasserabteil A unterteilt.

Um das Bilanzkammersystem 20 zum Füllen des Trockenkonzentratbehälters 100 verwenden zu können, wird das nach dem Füllen des Schlauchsystems im Altwasserteil A der Bilanzkammern vorhandene Flüssigkeitsvolumen durch das Schließen der Ventile V16 und V18 in den Zufuhrleitungen beider Bilanzkammern K1 und K2 als festes Pendelvolumen eingeschlossen. Das Pendelvolumen umfasst somit die Volumina der Altwasserabteile A sowie der sich zwischen diesen erstreckenden Leitung mit den darin angeordneten Ventilen V12 und V14. Das Pendelvolumen wird durch die geschlossenen Ventile V16 und V18 sowie V30 nach abgeschlossen.

Unter dem Begriff "Altwasserteil" ist der Teil A der Kammern zu verstehen, die während der Behandlung mit gebrauchter Dialyselösung vom Dialysator 200 beaufschlagt werden, sowie das diese Kammern verbindende Leitungsstück. In der Figur 1 ist das Pendelvolumen in den Kammern dunkel hinterlegt. Das Pendelvolumen wird im Rahmen der Behälterbefüllung zwischen den durch eine Leitung verbundenen Abteilen A der Kammern K1 und K2 hin und hergeschoben.

Die Ventile V12 und V14 in den Ausgangsleitungen der Kammern K1 und K2 sind offen, so dass das Pendelvolumen zwischen den Altwasserteilen der Kammern K1 und K2 hin und her bewegt werden kann. Um das Pendelvolumen nach außen abzuschließen, sind die Abflussleitungen durch Schließen der Ventile V30 und V19 abgesperrt.

Die Füllung des Behälters 100 erfolgt über die Frischwasserteile F der Kammern K1 und K2, d.h. über die Teile der Kammern, durch die während einer Dialysebehandlung frische Dialyselösung von einer Quelle zum Dialysator 200 bewegt wird. Die Frischwasserteile der Kammern K1 und K2 werden abwechselnd gefüllt und entleert. Sie sind in Figur 1 hell dargestellt.

In einer Phase wird durch Öffnen des Ventils V15 die Zufuhrleitung der Kammer K1 geöffnet und deren Abflussleitung mittels des Ventils V11 geschlossen. Gleichzeitig wird das Ventil V17 in der Zufuhrleitung der Kammer K2 geschlossen und deren Abflussleitung durch Öffnen des Ventils V13 geöffnet, so dass aus diesem Frischwasserteil der Kammer K2 Wasser über das offene Ventil VD1 durch die Leitung L1 in den Behälter 100 gefördert wird. Dazu strömt das Pendelvolumen in das Altwasserteil der Kammer K2. Gleichzeitig wird das Frischwassserteil der Kammer K1 durch die Leitung L2 mit Wasser gefüllt.

Bei einem neuen Zyklus, d.h. bei einer Bilanzkammerumschaltung wird das Ventil V17 geöffnet, das Ventil V13 geschlossen, das Ventil V15 geschlossen und das Ventil V11 geöffnet. Das Pendelvolumen strömt in das Altwasserteil der Kammer K1, so dass das in deren Frischwasserteil befindliche Wasser durch das offene Ventil VD1 und die Leitung L1 zu dem Behälter 100 gefördert wird, während gleichzeitig das Frischwasserteil der Kammer K2 durch die Leitung L2 mit Wasser gefüllt wird.

Die Pfeile in Figur 1 symbolisieren die Strömungsrichtung.

Das Pendelvolumen entspricht dem Volumen einer Bilanzkammer K1 bzw. K2, die ein identisches Volumen aufweisen.

Das Pendelvolumen legt das pro Bilanzkammerumschaltung bzw. das pro Zyklus geförderte Füllvolumen für den Trockenkonzentratbehälter 100 fest. Über die Anzahl der Umschaltungen wird das Gesamtfüllvolumen definiert, das dem Behälter 100 insgesamt zugeführt wird.

Der Trockenkonzentratbehälter 100 ist über das geöffnete Ventil S1 mit der Atmosphäre verbunden. Gleichwohl entsteht während der Befüllung des Behälters 100 mit Wasser aus dem Bilanzkammersystem 20 bedingt durch den Strömungswiderstand der Leitungen L1 bzw. Schläuche, durch die das Wasser von dem Bilanzkammersystem 20 zu dem Behälter 100 strömt, ein bestimmter Druckverlauf über die Zeit, wie beispielsweise Druckimpulse. Die Höhe dieser Druckimpulse, d.h. der jeweils gemessene Maximaldruck ist abhängig von der geförderten Flüssigkeitsmenge und von der Geometrie des Fluidsystems. Da der Absolutwert des Druckimpulses somit auch von der Geometrie und von der Compliance des Fluidsystems abhängt, wird bei dem Verfahren gemäß der Erfindung vorzugsweise die Höhe des Druckimpulses für jedes System, d.h. auch für jeden neuen Behälter fortlaufend gemessen.

Um die Messergebnisse nicht zu beeinflussen, sollte darauf geachtet werden, dass während des Füllvorgangs eines Behälters 100 der Strömungswiderstand zwischen Bilanzkammersystem 20 und dem Behälter 100 nicht verändert wird, d.h. Schlauchlängen, Rohrdurchmesser im Behälter etc. sollten konstant bleiben.

Zur Druckmessung dient in dem hier beschriebenen Ausführungsbeispiel ein Drucksensor S03, der wie aus Figur 1 ersichtlich über eine Leitung mit der Leitung L1 verbunden ist, die das Bilanzkammersystem 20 mit dem Behälter 100 verbindet.

Bei dem Behälter 100 kann es sich um einen Beutel oder auch um einen starren Behälter 100 handeln. In den Behälter können eine oder mehrere Leitungen hineinragen, durch die die Flüssigkeit zum Lösen des Feststoffes und ggf. Luft zur Verwirbelung und Verbesserung des Lösevorgangs eingeleitet wird.

Das Bezugszeichen 300 kennzeichnet die Leitung von einem Kompressor, der Luft in den Behälter 100 einleitet. Dazu sind die Ventile VA2 und VA1 geöffnet. Kommt es zu einer Unterbrechung des Füllvorgangs, werden diese Ventile geschlossen.

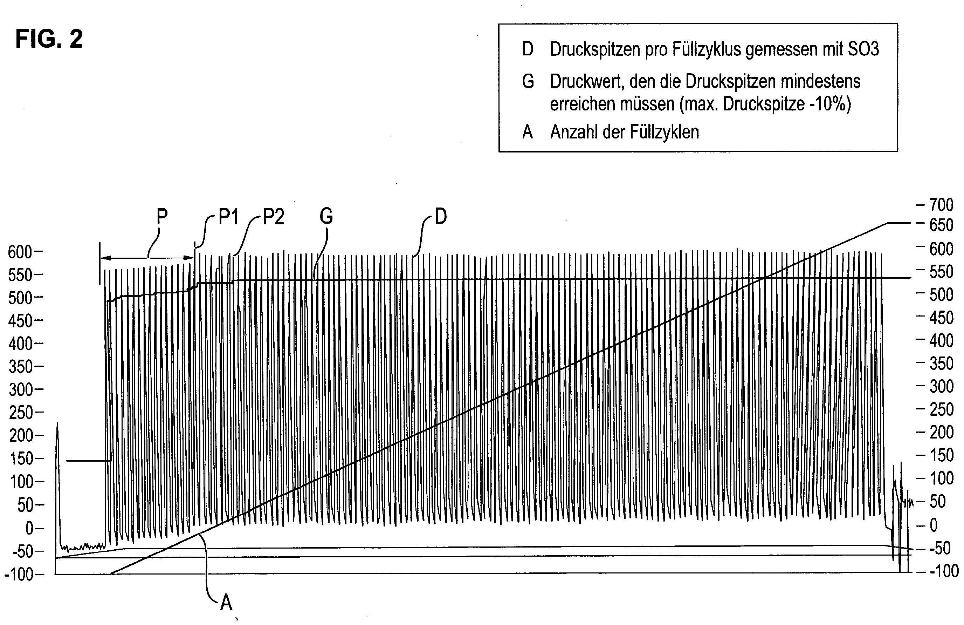

Die sich bei der Entleerung des Frischwasserteils und somit bei der Füllung des Behälters 100 ergebenden Druckspitzen sind in Figur 2 dargestellt. Wie dies aus Figur 2 ersichtlich ist, besteht der Druckverlauf aus sich wiederholenden Peaks, von denen jeder den Druckverlauf während eines Bilanzkammerzyklus, d.h. während des Bewegung eines Flüssigkeitsvolumen von einem Frischwasserteil der Kammern K1/K2 zum dem Behälter 100 darstellt. Die Peaks weisen jeweils einen Maximalwert und einen geringsten Wert auf.

Der obere Druckwert dient dazu, zu erkennen, ob die Bilanzkammer tatsächlich Flüssigkeit liefert. Mit dem unteren Druckwert wird erkannt, dass die Bilanzkammer komplett ausgedrückt wurde.

Die Druckspitzen bewegen sich zu Beginn des Füllvorgangs auf einem Plateau P, bis der Behälter, der vorzugsweise als Beutel ausgebildet ist, unter Druck steht. Anschließend kommt es zu einem leichten Anstieg der Druckspitzen, wie dies aus Figur 2 hervorgeht.

Als Grenzwert für den bei jedem Zyklus zumindest zu erreichenden Druck wird ein Druckwert 10 % geringer als die bis zu diesem Zyklus bislang erreichte Druckspitze verwendet. D.h. die bis dato erreichte größte Druckspitze eines Zyklus bildet die Grundlage für den Grenzwert, der in den folgenden Zyklen erreicht werden muss. Der jeweils geltende Grenzwert ist in Figur 2 mit dem Bezugszeichen G gekennzeichnet.

Wie dies aus Figur 2 hervorgeht, steigt in der Plateauphase P der Maximaldruck der Peaks an, so dass auch der Grenzwert G steigt. Ein weiterer Anstieg des Grenzwertes G ergibt sich bei Peak P1 sowie bei Peak P2, die die bis zu deren Auftreten höchsten Druckwerte aufweisen. Nach Peak P2 bleibt der Grenzwert konstant, da sich nach diesem Peak P2 keine höheren Drücke ergeben haben.

Der Verlauf A zeigt die Anzahl der Füllzyklen zur Befüllung des Behälters 100.

Wie dies aus Figur 2 hervorgeht, liegt in dem dargestellten Ausführungsbeispiel bei jedem Peak der Maximaldruck über dem Grenzwert G, so dass weder eine Alarmmeldung abgegeben wird noch ein Abbruch des Füllvorgangs erfolgt.

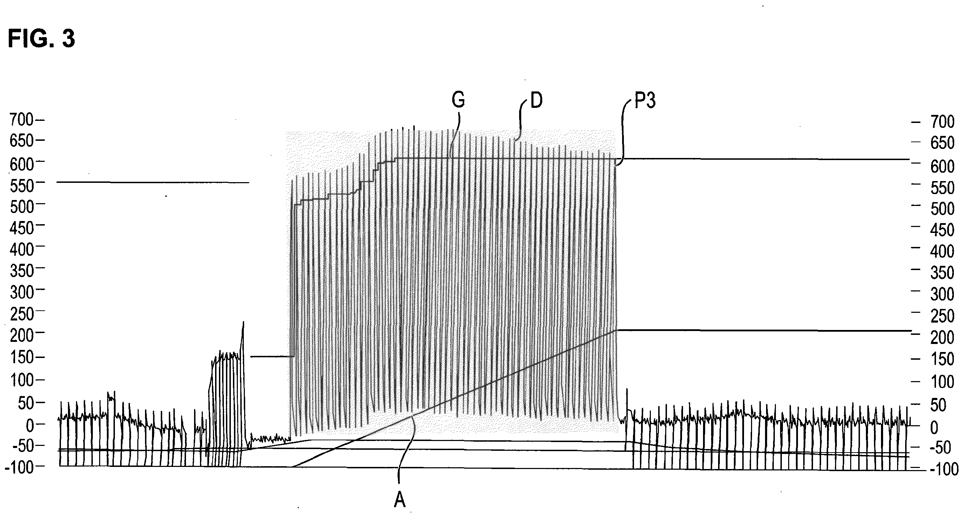

Figur 3 zeigt einen anderen Verlauf des Druckes, des Grenzwertes sowie der Anzahl der durchgeführten Zyklen. Bei dem Beispiel gemäß Figur 3 überschreitet der maximale Druckwert des Peaks P3 nicht den zu diesem Zeitpunkt geltenden Grenzwert G, was zur Folge hat, dass der Füllvorgang abgebrochen und ein Hinweissignal an den Nutzer abgegeben wird.

Das Nichterreichen des Grenzwertes G in dem Beispiel gemäß Figur 3 wird darauf zurückgeführt, dass aufgrund einer Undichtigkeit im System, wie beispielsweise durch eine Undichtigkeit der das Pendelvolumen abschließenden Ventile, das Pendelvolumen abnimmt, wodurch auch das direkt daran gekoppelte Füllvolumen abnimmt. Unterschreitet der Maximaldruck den Grenzwert, wird daraus geschlossen, dass das pro Bilanzkammerumschaltung geförderte Füllvolumen unter einen Grenzwert sinkt. Es wird Alarm ausgelöst und/oder der Füllvorgang gestoppt.

Mit der erfindungsgemäßen Vorgehensweise können Leckraten von ≥ 50 µl/Umschaltung detektiert werden. Wie oben ausgeführt, handelt es sich bei diesem Wert und bei den vorstehend genannten Werten um die Erfindung nicht beschränkende Beispiele.

## Patentansprüche

1. Verfahren zur Befüllung eines Behälters (100) enthaltend ein Konzentrat, das derart ausgebildet ist, dass es bei seiner Lösung in oder bei seiner Verdünnung mit einer Flüssigkeit, vorzugsweise Wasser, ein Flüssigkonzentrat oder einen Teil eines Flüssigkonzentrats bildet, das zur Herstellung einer Dialyselösung geeignet ist, wobei
die Befüllung des Behälters (100) mittels des Bilanzkammersystems (K1, K2) eines Dialysegerätes erfolgt, das mehrere Kammern (K1, K2) aufweist, aus der die Flüssigkeit in Form von sich wiederholenden Zyklen in den Behälter (100) gefördert wird,
**dadurch gekennzeichnet, dass**
der zeitliche Verlauf des Druckes, unter dem die von dem Bilanzkammersystem zu dem Behälter gelangende Flüssigkeit steht, während eines Zyklus der Füllung des Behälters (100) gemessen wird, und
ein Alarmsignal ausgegeben wird und/oder der Füllvorgang des Behälters (100) gestoppt wird, wenn der gemessene Maximaldruck (D) in einem Zyklus einen Grenzwert (G) nicht übersteigt.

2. Verfahren nach Anspruch 1, **dadurch gekennzeichnet, dass** das Bilanzkammersystem (K1, K2) des Dialysegerätes wenigstens zwei Kammern (K1, K2) aufweist und dass ein Pendelvolumen zwischen den beiden Kammern (K1, K2) hin und her strömt, wobei vorzugsweise vorgesehen ist, dass das Pendelvolumen dem Volumen einer Kammer (K1, K2) entspricht.

3. Verfahren nach Anspruch 1 oder 2, **dadurch gekennzeichnet, dass** der Grenzwert (G) von dem Maximaldruck abhängt, der in wenigstens einem der vorhergegangenen Zyklen gemessen wurde oder der vorgegeben ist.

4. Verfahren nach Anspruch 3, **dadurch gekennzeichnet, dass** der Grenzwert (G) um einen bestimmten Prozentsatz oder um einen bestimmten Absolutwert unter dem höchsten, in den vorhergegangenen Zyklen gemessenen Maximaldruck liegt.

5. Verfahren nach einem der vorhergehenden Ansprüche, **dadurch gekennzeichnet, dass** der Grenzwert (G) im Laufe des Füllvorgangs nicht abgesenkt wird, insbesondere dann nicht, wenn der gemessene Maximaldruck in einem Zyklus geringer ist als in einem vorherigen Zyklus.

6. Verfahren nach einem der vorhergehenden Ansprüche, **dadurch gekennzeichnet, dass** der Druck in der Leitung zwischen den Kammern (K1, K2) und dem Behälter (100) oder in einer damit in Verbindung stehenden Leitung gemessen wird.

7. Verfahren nach einem der vorhergehenden Ansprüche, **dadurch gekennzeichnet, dass** der Druck bei einer Entleerung einer Kammer zunächst ansteigt und dann abfällt und dass ein Alarmsignal ausgegeben wird und/oder der Füllvorgang des Behälters gestoppt wird, wenn ein unterer Grenzwert für den Druck nicht innerhalb eines bestimmten Zeitfensters seit Zyklusbeginn erreicht oder unterschritten wird.

8. Verfahren nach Anspruch 7, **dadurch gekennzeichnet, dass** das Zeitfenster für den ersten oder für die ersten Zyklen länger ist als für die folgenden Zyklen.

9. Verfahren nach einem der vorhergehenden Ansprüche, **dadurch gekennzeichnet, dass** der erste oder die ersten Zyklen eines Füllvorgangs und/oder der letzte Zyklus vor einer Unterbrechung des Füllvorgangs nicht überwacht werden.

10. Verfahren nach einem der vorhergehenden Ansprüche, **dadurch gekennzeichnet, dass** der Behälter (100) während des Füllvorgangs zur Atmosphäre hin belüftet ist.

11. Verfahren nach einem der vorhergehenden Ansprüche, **dadurch gekennzeichnet, dass** der Grenzwert (G) nur zurückgesetzt wird, wenn bestätigt oder erkannt wird, dass ein neuer Behälter (100) auf das Dialysegerät aufgesteckt wurde.

12. Verfahren nach einem der vorhergehenden Ansprüchen, **dadurch gekennzeichnet, dass** es sich bei dem in dem Behälter (100) befindlichen Konzentrat um eine Flüssigkeit und/oder um ein Trockenkonzentrat, insbesondere um ein saures Trockenkonzentrat handelt.

13. Verfahren nach einem der vorhergehenden Ansprüche, **dadurch gekennzeichnet, dass** während der Füllung des Behälters (100) mit Flüssigkeit ein Gas, vorzugsweise Luft in den Behälter (100) eingeleitet wird.

14. Verfahren nach einem der vorhergehenden Ansprüche, **dadurch gekennzeichnet, dass** bei Erreichen eines unteren Grenzwertes für den Druck nur dann ein neuer Zyklus eingeleitet wird, wenn eine bestimmte Mindestzeitspanne seit Zyklusbeginn verstrichen ist.

15. Dialysegerät mit Mitteln zur Befüllung eines Behälters (100) vorzugsweise enthaltend wenigstens ein Konzentrat, wobei das Konzentrat derart ausgebildet ist, dass es bei seiner Lösung oder bei seiner Verdünnung in/mit einer Flüssigkeit, vorzugsweise Wasser, wenigstens ein Flüssigkonzentrat oder einen Teil eines Flüssigkonzentrats bildet, das zur Herstellung wenigstens einer Dialyselösung geeignet ist, und wobei die Mittel zur Befüllung des Behälters (100) wenigstens ein Bilanzkammersystem (K1, K2) des Dialysegerätes umfassen, das mehrere Kammern (K1, K2) aufweist, aus der die Flüssigkeit in Form von wiederholten Zyklen in den Behälter (100) bewegt wird, **dadurch gekennzeichnet, dass** das Dialysegerät wenigstens eine Steuer- oder Regelungseinheit aufweist, die ausgebildet ist, das Verfahren gemäß einem der Ansprüche 1 bis 14 durchzuführen.

## Claims

1. A method of filling a container (100) containing a concentrate which is formed such that it forms a liquid concentrate or a part of a liquid concentrate on its solution in or its dilution with a liquid, preferably water, said liquid concentrate or part of a liquid concentrate being suitable for preparing a dialysis solution,
with the filling of the container (100) taking place by means of the balance chamber system (K1, K2) of a dialysis machine which has a plurality of chambers (K1, K2) from which the liquid is conveyed into the container (100) in the form of repeating cycles,
**characterized in that**
the time development of the pressure the liquid moving from the balance chamber system to the container is subjected to is measured during a cycle of the filling of the container (100); and
an alarm signal is emitted and/or the filling procedure of the container (100) is stopped if the measured maximum pressure (D) in a cycle does not exceed a limit value (G).

2. A method in accordance with claim 1, **characterized in that** the balance chamber system (K1, K2) of the dialysis machine has at least two chambers (K1, K2); and **in that** an oscillating volume flows to and fro between the two chambers (K1, K2), with provision preferably being made that the oscillating volume corresponds to the volume of a chamber (K1, K2).

3. A method in accordance with claim 1 or claim 2, **characterized in that** the limit value (G) depends on the maximum pressure which was measured or which is predefined in at least one of the preceding cycles.

4. A method in accordance with claim 3, **characterized in that** the limit value (G) lies below the highest maximum pressure measured in the preceding cycles by a specific percentage or by a specific absolute value.

5. A method in accordance with one of the preceding claims, **characterized in that** the limit value (G) is not lowered in the course of the filling process, in particular not when the measured maximum pressure is smaller in one cycle than in a previous cycle.

6. A method in accordance with one of the preceding claims, **characterized in that** the pressure is measured in the line between the chambers (K1, K2) and the container (100) or in a line in communication therewith.

7. A method in accordance with one of the preceding claims, **characterized in that** the pressure first rises on an emptying of a chamber and then drops; and **in that** an alarm signal is emitted and/or the filling process of the container is stopped when a lower limit value for the pressure is not reached or is not fallen below within a specific time window since the cycle start.

8. A method in accordance with claim 7, **characterized in that** the time window for the first cycle or cycles is longer than for the following cycles.

9. A method in accordance with one of the preceding claims, **characterized in that** the first cycle or cycles of a filling process and/or the last cycle is not monitored before an interruption of the filling process.

10. A method in accordance with one of the preceding claims, **characterized in that** the container (100) is vented to the atmosphere during the filling process.

11. A method in accordance with one of the preceding claims, **characterized in that** the limit value (G) is only reset when it is confirmed or recognized that a new container (100) has been placed onto the dialysis machine.

12. A method in accordance with one of the preceding claims, **characterized in that** the concentrate located in the container (100) is a liquid and/or a dry concentrate, in particular a dry acid concentrate.

13. A method in accordance with one of the preceding claims, **characterized in that** a gas, preferably air, is introduced into the container (100) during the filling of the container (100) with liquid.

14. A method in accordance with one of the preceding claims, **characterized in that** a new cycle is only initiated once a lower limit value for the pressure has been reached if a specific minimum length of time has elapsed since the cycle start.

15. A dialysis machine having means for filling a container (100), preferably containing at least one concentrate, with the concentrate being formed such that it forms at least one liquid concentrate or a part of a liquid concentrate on its solution in or its dilution with a liquid, preferably water, said liquid concentrate or part of a liquid concentrate being suitable for preparing at least one dialysis solution, and with the means for filling the container (100) comprising at least one balance chamber system (K1, K2) of the dialysis machine which has a plurality of chambers (K1, K2) from which the liquid is moved into the container (100) in the form of repeated cycles, **characterized in that** the dialysis machine has at least one control or regulation unit which is configured to carry out the method in accordance with one of the claims 1 to 14.

## Revendications

1. Procédé de remplissage d'un récipient (100) contenant un concentré, qui est conçu de telle manière que, lors de sa dissolution dans ou lors de sa dilution avec un liquide, de préférence de l'eau, il forme un concentré liquide ou une partie d'un concentré liquide, qui est adapté pour produire une solution de dialyse,
le remplissage du récipient (100) étant effectué au moyen du système de chambres d'équilibrage (K1, K2) d'un appareil de dialyse, qui comporte plusieurs chambres (K1, K2), à partir desquelles le liquide est transporté dans le récipient (100) par cycles répétitifs,
**caractérisé en ce que**,
l'évolution dans le temps de la pression, à laquelle se trouve le liquide passant du système de chambres d'équilibrage au récipient, est mesurée pendant un cycle du remplissage du récipient (100), et
un signal d'alarme est émis et/ou le procédé de remplissage du récipient (100) est arrêté quand la pression maximale (D) mesurée ne dépasse pas une valeur limite (G) pendant un cycle.

2. Procédé selon la revendication 1, **caractérisé en ce que** le système de chambres d'équilibrage (K1, K2) de l'appareil de dialyse comporte au moins deux chambres (K1, K2) et **en ce qu'**un volume oscillant entre les deux chambres (K1, K2) circule dans les deux sens, le volume oscillant étant de préférence prévu correspondant au volume d'une chambre (K1, K2).

3. Procédé selon la revendication 1 ou 2, **caractérisé en ce que** la valeur limite (G) dépend de la pression maximale, qui a été mesurée dans au moins un des cycles précédents ou qui est prédéfinie.

4. Procédé selon la revendication 3, **caractérisé en ce que** la valeur limite (G) est d'un pourcentage défini ou d'une valeur absolue définie inférieure à la pression maximale la plus élevée mesurée dans les cycles précédents.

5. Procédé selon l'une des revendications précédentes, **caractérisé en ce que** la valeur limite (G) n'est pas diminuée au cours du procédé de remplissage, notamment pas quand la pression maximale mesurée dans un cycle est inférieure à celle d'un cycle précédent.

6. Procédé selon l'une des revendications précédentes, **caractérisé en ce que** la pression est mesurée dans le conduit entre les chambres (K1, K2) et le récipient (100) ou dans un conduit en liaison avec celui-ci.

7. Procédé selon l'une des revendications précédentes, **caractérisé en ce que**, lors d'un vidage d'une chambre, la pression augmente d'abord puis diminue et **en ce qu'**un signal d'alarme est émis et/ou le procédé de remplissage du récipient est arrêté quand la pression n'atteint pas ou ne passe pas en dessous d'une valeur limite inférieure pendant un intervalle de temps défini depuis le début du cycle.

8. Procédé selon la revendication 7, **caractérisé en ce que** l'intervalle de temps pour le premier ou pour les premiers cycles est plus long que pour les cycles suivants.

9. Procédé selon l'une des revendications précédentes, **caractérisé en ce que** le premier ou les premiers cycles d'un procédé de remplissage et/ou le dernier cycle ne sont pas surveillés avant une interruption du procédé de remplissage.

10. Procédé selon l'une des revendications précédentes, **caractérisé en ce que** le récipient (100) est en liaison avec l'atmosphère pendant le procédé de remplissage.

11. Procédé selon l'une des revendications précédentes, **caractérisé en ce que** la valeur limite (G) n'est réinitialisée qu'en cas de branchement confirmé ou détecté d'un nouveau récipient (100) sur l'appareil de dialyse.

12. Procédé selon l'une des revendications précédentes, **caractérisé en ce que** le concentré se trouvant dans le récipient (100) est un liquide et/ou un concentré sec, en particulier un concentré sec acide.

13. Procédé selon l'une des revendications précédentes, **caractérisé en ce que**, pendant le remplissage du récipient (100) avec du liquide, un gaz, de préférence de l'air, est introduit dans le récipient (100).

14. Procédé selon l'une des revendications précédentes, **caractérisé en ce que**, lorsque la pression atteint une valeur limite inférieure, un nouveau cycle est commencé uniquement si un laps de temps minimal défini depuis le début du cycle s'est écoulé.

15. Appareil de dialyse comprenant des moyens de remplissage d'un récipient (100), contenant de préférence au moins un concentré, le concentré étant conçu de telle manière que, lors de sa dissolution ou lors de sa dilution dans/avec un liquide, de préférence de l'eau, il forme au moins un concentré liquide ou une partie d'un concentré liquide, qui est adapté pour produire au moins une solution de dialyse, et les moyens de remplissage du récipient (100) comprenant au moins un système de chambres d'équilibrage (K1, K2) de l'appareil de dialyse, qui comporte plusieurs chambres (K1, K2), à partir desquelles le liquide est déplacé dans le récipient (100) par cycles répétitifs, **caractérisé en ce que** l'appareil de dialyse comporte au moins une unité de commande ou de régulation, qui est conçue pour exécuter le procédé selon l'une des revendications 1 à 14.
